# EUROPEAN PATENT APPLICATION

(11) **EP 4 151 736 A1**
(43) Date of publication of application: **22.03.2023**
(21) Application number: 21198072.7
(22) Date of filing: 21.09.2021
(51) Int. Cl.: C12N 15/86, C12Q 1/6897

(54) **NOVEL SYSTEM FOR THE QUANTIFICATION OF ANTI-AAV NEUTRALIZING ANTIBODIES**

(71) Applicant: SVAR Life Science AB, 202 11 Malmö (SE)
(72) Inventor: TOVEY, Michael, 75005 PARIS (FR); VALLETTE, Benoit, 94800 VILLEJUIF (FR); LALLEMAND, Christophe, 75018 PARIS (FR)
(74) Representative: Zacco Denmark A/S

(57) **Abstract**

The present invention relates to a novel system for the detection and quantification of neutralizing antibodies directed against either adeno-associated virus (AAV) or recombinant AAV vectors with improved sensitivity and specificity.

## Description

### Field of the invention

The present invention relates i.a. to a novel two-component system for the detection and quantification of neutralizing antibodies directed against recombinant adeno-associated virus (AAV) vectors. The invention comprises:
(i). a packing cell line for the production of the AAV serotype or the recombinant AAV vector to which antibodies are to be detected and that contains within the virus genome or within the transgene construct a tag sequence and
(ii). a reporter-gene cell line incorporating a reporter gene-promoter construct that responds specifically to the tag sequence and a method of using the same for detecting and optimally quantitating anti-AAV neutralizing antibodies directed against the capsid, the virus genome, and/or the transgene or transgene product in a test sample(s). The invention further relates to a method for high-throughput screening for detecting and optimally monitoring the immune response to recombinant AAV vectors with optimal sensitivity, signal strength, and biological fidelity. In a broader sense, the invention also relates to use of the packaging cell line in combination with the reporter-gene cell line in diagnostics.

### Background of the invention

Recombinant adeno-associated virus (AAV) vectors are used to treat an increasing number of genetic disorders and although for the most part AAV based gene therapy is well tolerated, the immune response to either the AAV capsid, AAV genome, transgene, or transgene product is associated with a reduced level, or complete loss of expression of the transgene, and hence a loss of efficacy (1). The development of neutralizing antibodies against recombinant AAV vectors is often related to previous exposure to one or more AAV serotypes usually in early childhood and the high degree of cross reactivity of anti-AAV antibodies across different AAV serotypes (1,2).

AAV is a single-stranded DNA virus, the genome of which consists of three genes *rep, cap,* and *aap*, flanked by 145 bp inverted terminal repeats (ITRs), the first 125 nucleotides of which is a palindromic sequence that folds upon itself to form a T-shaped hairpin loop secondary structure. The ITRs act as a primer for second-strand synthesis and are required in cis for virus replication or expression of a transgene (3). The *rep* gene encodes 4 non-structural proteins, Rep78, Rep68, Rep52, and Rep40 encoded by a single ORF using two different promoters, p5 for Rep78/68 and p19 for Rep52/40, and alternative splicing. The Rep78 and Rep68 proteins bind to the ITRs and are required for genome replication while Rep52 and Rep40 are responsible for packaging the viral genome into capsids during virus replication. The Rep proteins also play a role in site-specific genome integration. The cap gene encodes 3 structural proteins Vp1, Vp2, and Vp3, transcribed from the same ORF under control of the p40 promoter using alternative start codons and alternative splicing, and are assembled at a 1:1:10 ratio to form the virus capsid (4). The *Aap* gene encodes the Assembly-Activated Protein (AAP) from an alternative reading frame within the *Cap* ORF. AAP localizes AAV capsid proteins to the nucleolus and assembles the three Cap proteins into the 60-mer icosahedral viral capsid (3).

Recombinant AAV vectors devoid of the *rep, cap,* and *aap* genes are limited to a size of approximately 4.8 kb and consist of a suitable promoter, that may be constitutive or tissue-specific depending upon the intended application, the transgene, and a poly A tail contained within the ITRs. AAVs are replication defective and require a helper virus, usually adenovirus (5), to supply the early helper proteins required for virus replication or for the expression of a transgene. Herpes simplex virus (HSV), and to some extent vaccinia virus, can substitute for the presence of adenovirus for the supply of the early helper proteins (5, 6). The early genes required for efficient AAV vector production can, however, also be supplied by transfecting a suitable packaging cell line with plasmids expressing the helper proteins in addition to plasmids encoding the transgene-promoter construct and the *rep, cap* and *aap* genes (7).

The capsid plays a key role in serotype-specific virus-cell interactions initially by binding to carbohydrates on the cell surface. Thus, AAV serotypes 2,3, & 6 bind heparin sulfate proteoglycan, while AAV1, 4, 5, & 6 bind sialic acid and AAV9 binds galactose (8). Most AAV serotypes then interact with differing moieties of a novel AAV receptor (AAVR) required for cellular internalization (9). An additional highly conserved G protein-coupled receptor like protein GPR108, localized in the *trans-Golgi,* has recently been identified that is required for the entry of all AAV serotypes except for the highly divergent AAV5 serotype that is AAVR dependent but GPR108-independent (10). Thus, all AAV serotypes identified to date require either AAVR or GPR108 or both AAVR and GPR108 for cellular transduction (9,10).

Although AAV infection is not associated with any disease in humans or other mammals the efficacy of AAV mediated gene-therapy is limited by the development of anti-AAV antibodies, often related to a previous exposure to AAV and the high degree of cross-reactivity between different serotypes, leading to a reduced level or complete loss of expression of the transgene, and hence a loss of efficacy. Cell-based assays based on the use of a reporter AAV vector that is incubated with the test sample prior to *in vitro* transduction of a cell line, the so called transduction inhibition assay, often require high multiplicities of infection (MOIs) resulting in a low degree of sensitivity, are subject to non-specific effects, and are at best a surrogate of the immune response to the actual recombinant AAV vector to which the antibody response is to be determined. There is a need therefore to develop improved methods with increased sensitivity and specificity for the detection and quantification of the neutralizing antibody response to both AAV infection and treatment with recombinant AAV vectors.

### Summary of the invention

The present invention was made in view of the prior art described above, and the object of the present invention is to provide a novel system for the detection and quantification of neutralizing antibodies directed against recombinant adeno-associated virus (AAV) vectors with improved sensitivity and specificity.

In order to obtain the above technical effects and provide for much improved methods and cell lines in relation thereto, present invention relates i.a. to a cell line. This cell line may be denoted as a packaging cell line and may also be referred to a first component or Component 1, all used interchangeably herein. The packaging cell line may comprise one or more different tag sequences.

Moreover, and in another aspect, the present invention relates to a reporter-gene cell line incorporating a reporter-gene promoter construct that responds specifically to the one or more tag sequences of the packaging cell line. The reporter-gene cell line may be regarded as second component or referred to as Component 2, all used interchangeably herein.

In one aspect of the invention, component 1 and component 2 may function in concert or may be used in combination with each other.

In a further aspect, the present invention relates to a method of using component 1 and component 2 for detecting and optimally quantitating neutralizing antibodies against recombinant AAV vectors in a test sample. Specifically, the invention relates to use of component 1 and component 2 for an improved sensitivity and specificity for the detection and quantification of the neutralizing antibody response to treatment with recombinant AAV vectors. In another aspect, the invention relates to a two-component system comprising a packing cell line **(Component 1)** for the production of the AAV serotype or the recombinant AAV vector, to which the antibody response is to be determined, that contains a tag sequence within the virus genome or the transgene construct, and a reporter-gene cell line **(Component 2)** incorporating a reporter-gene promoter construct that responds specifically to the tag sequence of Component 1 and a method of using the same for detecting and optimally quantitating neutralizing antibodies against recombinant AAV vectors in a test sample. The invention further relates to a method for high-throughput screening for detecting and monitoring the immune response to recombinant AAV vectors with optimal sensitivity, signal strength, and biological fidelity.

With respect to the tag sequence of present invention, cell lines disclosed herein may comprise one or more tag sequences. In the case of the presence of several tag sequences, the tag sequences may be identical or may different. In another aspect, and in the case where several tag sequences are present, a group of tag sequences may be identical, while the remaining tag sequences may be different.

In one aspect, the tag sequence may be e.g. a tag sequence contained within the AAV genome or e.g. the transgene-promoter construct is the Gal4

DNA binding domain, or the tag sequence contained within the AAV genome or the transgene-promoter construct encoding a cGMP specific receptor protein (CRP), or e.g. the tag sequence contained within the AAV genome or the transgene construct encoding the trans-silencer VanR fused to the transactivator VP16.

As is apparent from the above, and in the aspect that Component 1 is used in combination with Component 2, the reporter-gene cell line incorporating a reporter-gene promoter construct will responds specifically to the one or more tag sequences of the packaging cell line, such that the reporter-gene promotor construct responds to each same or different tag sequence in Component 1.

### Component 1: Packaging Cell Line.

In one aspect, present invention relates to a packing cell line is established in which the constituent components required for the expression of the serotype of the recombinant AAV vector are supplied by transient transfection or preferably have been partially or completely integrated into the genome of the chosen cell line. The chosen cell line may be referred to as a host cell line.

The choice of host cell line is determined by its safety profile for either the analysis of biological samples and for the ability of the cells to tolerate the toxicity of the AAV Rep protein following regulated expression of the rep gene and E4orf6 (11). Such cell lines include, but are not limited to, the human lung adenocarcinoma cell line A549 (ATCC catalogue # CCL-185) or the human embryonic kidney cell line HEK293T (ATCC catalogue # ACS-4500).

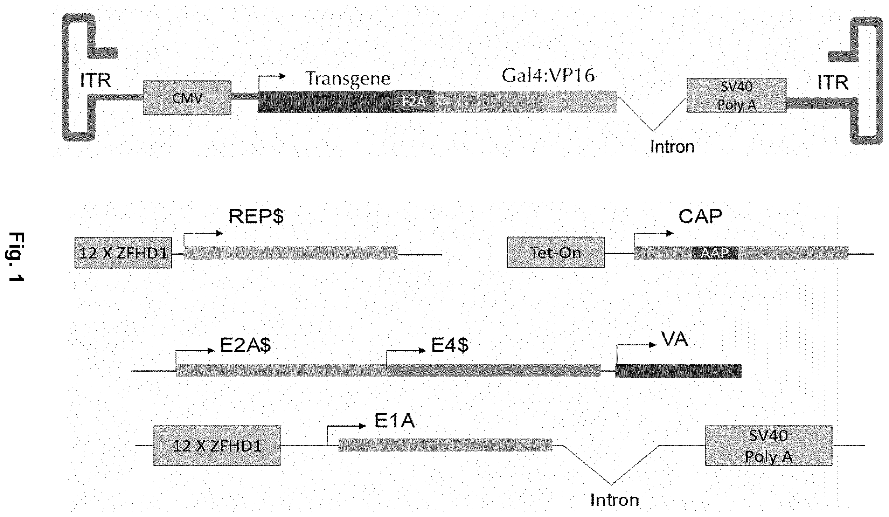

The chosen cell line (host cell line) is transfected with the transgene of choice under the control of a suitable promotor. The promotor may in principle be any suitable promotor known in the art. In one aspect, the promotor may be an inducible promoter. In another aspect, the promoter may be either a constitutive promoter, such as the cytomegalovirus (CMV) immediate early promoter, or an appropriate tissue-specific promoter regulating expression of the transgene, chosen as a function of the desired tissue-specific expression of the transgene, and a suitable polyadenylation site such as that from SV40 or an alternative polyadenylation site, contained within the ITRs of AAV2 (as illustrated in Figure 1). Importantly, the cell lines of Component 1 comprises a tag sequence within the ITRs to monitor a specific recombinant AAV vector of choice.

The host cells may further be co-transfected with the *cap* gene under the control of a constitutive promoter such as the CMV minimal promoter (as illustrated in Figure 1). The host cells may be co-transfected with the *cap* and *rep* gene on two independent plasmids since expression of the *cap* and *rep* genes on separate plasmids has been reported to increase AAV vector production (12).

The gene encoding E1 and the E1A and E1B ORFs, is not expressed in A549 cells in contrast to HEK293T cells. Thus, A549 cells were transfected with the *E1A* gene, expressed under the control of a suitable inducible promoter to prevent the E1a protein activating the *Rep, E2* and *E4* genes leading to cellular toxicity. Thus, in Example 1 the *rep* and *E1* genes are expressed under the control of a rapamycin-inducible promoter consisting of a 12-fold tandem repeat of the DNA binding domain ZFHD1 fused to the FK506 binding protein (FKBP). The FKBP-rapamycin-associated protein (FRAP) is fused to the VP16 trans-activator from HSV-1 such that addition of rapamycin will induce the formation of hetero-dimers between FKBP and FRAP leading to a tightly controlled expression of the gene encoding the E1 early protein.

Controlled expression of Rep78/68 has also been shown to increase the level of AAV vector production (12). Although transfection of HEK293 cells with *E4 orf6* alone has been reported to be sufficient for adenovirus-free production of a recombinant AAV vector (13) the early proteins E2A, E4, and VA RNA have been reported to be required for efficient production of recombinant AAV (14). Thus, in one aspect, the packaging cell line may be co-transfected with expression vectors expressing E2A, E4, and VA RNA.

### Component 2: Reporter Cell Line.

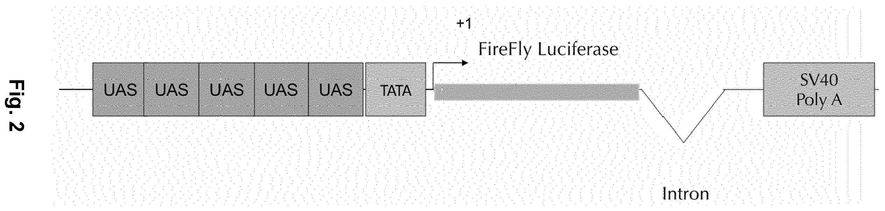

In one aspect, the present invention also relates to a reporter-gene cell line that has been developed that responds specifically to the tag sequence (the tag sequence within the ITRs to monitor a specific recombinant AAV vector of choice in Component 1) contained within the AAV genome or transgene construct such that contact of the reporter cells with either a virus preparation, or packaging cell line producing virus expressing the tag sequence within the genome of the AAV serotype or recombinant AAV vector to which neutralizing antibodies are to be quantified will result in activation of the reporter-gene expressed by the reporter cells. It is understood that in order to increase sensitivity the reporter-gene cell line can be stably transfected with either the cell surface AAV receptor alone (9), or the endosomal AAV receptor alone (10), or both the cell surface receptor and the endosomal AAV receptors. In **Example 1** the packaging cell line expresses a transgene-promoter construct containing a tag sequence encoding the Gal4 DNA binding domain, that will bind specifically to the Gal4 upstream activation sequence (UAS) regulating expression of the firefly luciferase (FL) gene in the Reporter cells. Specifically, in Example 1 the AAV-2 responsive reporter cell line contains a 5-fold tandem repeat of the Gal 4 UAS, regulating expression of the FL reporter-gene construct in a HEK293 host cell (Figure 2).

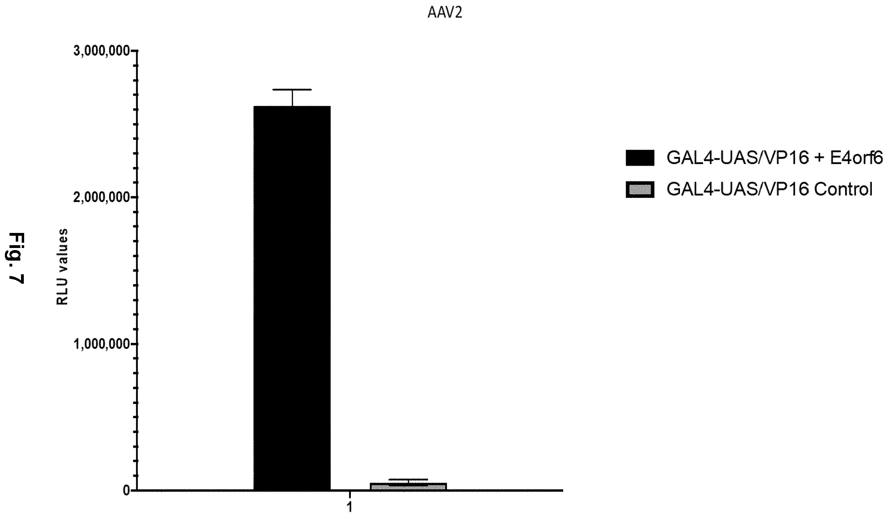

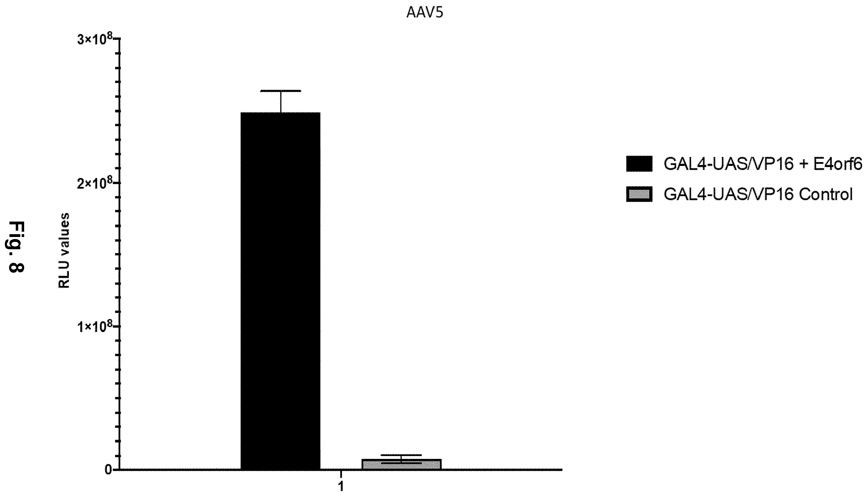

In a preferred embodiment the reporter-cell line (Component 2) is transected either transitorily or stably with the immediate early enhancer protein E4orf6 that significantly increases GAL4-UAS regulated firefly luciferase expression when incubated with packaging cells (Component 1) containing a *cap* gene encoding either the AAV2 or AAV5 cap proteins (Figures 7 & 8).

### Interaction of the packaging cell line and the reporter cells

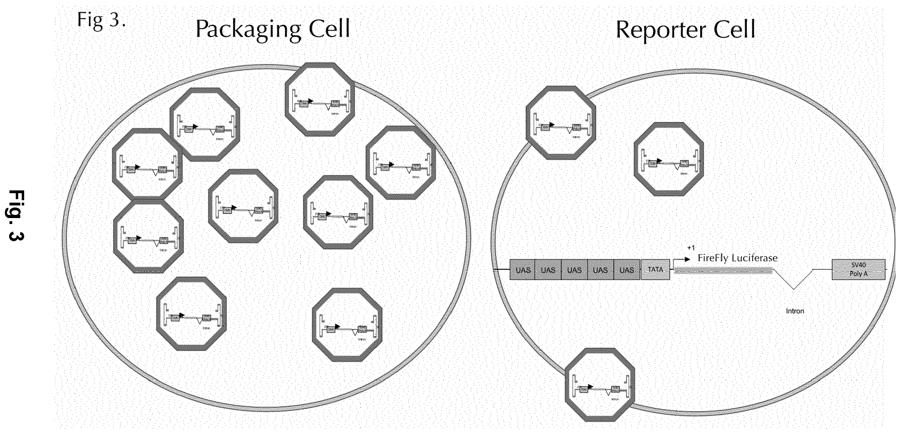

In one aspect, the present invention relates to component 1 and component 2 in combination such that the two cell-lines interact with each other (Figures 3 to 5). Thus, in one aspect the present invention relates to a method, the method comprising the steps of:
(a). contacting the virus produced by the packaging cells according to the present invention with the sample(s) to be tested for various times and at various temperatures comprising or including any one of the temperatures +4, 20, or 37°C, or any temperature in the interval of from e.g. about +4°C to about 37°C, prior to incubation of the virus preparation and the sample(s) with the reporter-cells at about 37°C for various times preferably for about 18 hours or more prior to quantification of FL reporter-gene activity using a suitable substrate such as the commercially available substrate Bright-Glo (Promega, Madison, WI), or any other suitable substrate enabling detection by any means,
(b). contacting the virus-producing packaging cells with the sample(s) to be tested for various times at and at various temperatures comprising or including any one of the temperatures +4, 20, or 37°C, or any temperature in the interval of from e.g. about +4°C to about 37°C, prior to incubation of the packaging cell line and the sample(s) with the reporter-cells at 37°C for various times preferably for about 6 to about 18 hours or more prior to quantification of FL reporter-gene activity using a suitable substrate such as e.g. the commercially available substrate Bright-Glo (Promega, Madison, WI), or any other suitable substrate enabling detection by any means,
(c). contacting the virus-producing packaging cell line with the sample(s) to be tested and the reporter-cells and incubating directly at about 37°C for various times preferably for about 18 hours or more prior to quantification of FL reporter-gene activity using a suitable substrate such as e.g. the commercially available substrate Bright-Glo (Promega, Madison, WI), or any other suitable substrate enabling detection by any means,
(d). freezing the virus-producing packaging cell line together with the reporter cells at the appropriate cell concentrations in a suitable cryo-protective medium, thawing the cells, contacting the packaging cells/reporter-gene cells with the sample(s) to be tested and incubating the sample(s), packaging cells-reporter-cells at about 37°C for various times preferably for about 18 hours or more prior to quantification of FL reporter-gene activity using a suitable substrate such as the commercially available substrate Bright-Glo (Promega, Madison, WI), or any other suitable substrate enabling detection by any means,
(e). in a preferred embodiment freezing the virus-producing packaging cell line and the reporter cells separately at the appropriate cell concentrations in a suitable cryo-protective medium, thawing the cells, contacting the virus-producing packaging cell line with the sample(s) to be tested and incubation of the virus-producing packaging cells and the sample(s) with the reporter-cells at about 37°C for various times preferably for about 6 to about 18 hours or more prior to quantification of FL reporter-gene activity using a suitable substrate such as the commercially available substrate Bright-Glo (Promega, Madison, WI), or any other suitable substrate enabling detection by any means. The use of frozen thaw-and-use virus producing packaging cells obviates the need for the end user to produce the particular AAV serotype or recombinant AAV vector to which neutralizing antibodies are to be detected and contacting the virus-producing packaging cell line with the samples(s) to be tested obviates the need to extract and purify the challenge virus.

The cell-free recombinant AAV vector or the AAV recombinant vectorproducing packaging cells interact with the reporter-gene cell line resulting in virus uptake, internalization, and AAV-serotype specific activation of the FL reporter-gene. The initial interaction of the cell-free challenge virus vector or AAV vector producing packaging cells with the reporter cells is most probably mediated by the release of virus and interaction of the capsid with carbohydrates on the cell surface in a serotype-specific interaction. Thus, AAV serotypes 2, 3, & 6 are known to bind to heparin sulfate proteoglycan, while AAV serotypes 1,4, 5, & 6 bind sialic acid and AAV9 binds to galactose (8). The AAV serotypes then interact with either the AAV receptor required for cellular internalization (9) and/or the novel G protein-coupled receptor like protein GPR108, localized in the *trans-Golgi* (10). In one embodiment, the reporter gene encodes an enzyme. In a preferred embodiment, the reporter is a luciferase, such as firefly luciferase, Renilla luciferase, Metridia luciferase, or a novel luciferase present in solution as soluble active monomers, or as fusion proteins with other proteins including luciferases or fluorescent proteins such as green fluorescent proteins (GFPs) enhanced green fluorescent protein (EGFP), red fluorescent proteins (RFPs), yellow fluorescent protein (YFP), blue fluorescent protein (BFP) and variants thereof displaying a different excitation/emission spectra, horseradish peroxidase (HRP), or various conjugates thereof, secreted human placental alkaline phosphatase (SEAP), or various conjugates thereof, chloramphenicol acetyltransferase (CAT), or various linker proteins, or attached to solid surfaces such as particles, beads, assay-plates or tubes.

In another embodiment, the reporter gene encodes a fluorescent protein. Useful fluorescent protein includes green fluorescent protein (GFP) and related fluorescent protein, e.g. enhanced green fluorescent protein (EGFP), red fluorescent protein (RFP), yellow fluorescent protein (YFP), blue fluorescent protein (BFP) and variants there of displaying a different excitation/emission spectra.

In a further embodiment the reporter-cells may be co-transfected with a first reporter-gene such as the firefly luciferase (FL) reporter-gene under the control of a chimeric promoter that responds specifically to the tag sequence present within the AAV genome or the recombinant AAV encoded transgene promoter construct contained within the AAV ITRs and a second reporter-gene such as the Renilla luciferase (RL) under the control of a constitutive promoter that allows the tag sequence activated FL activity to be normalized with respect to the constitutive level of expression of RL activity rendering the assay results independent of cell number and providing a means for compensating for inter-sample variation in cell densities due to loss of cells or variation in the number of cells seeded. The ability to normalize AAV-activated FL activity relative to the constitutive express of RL activity also provides a means to compensate for serum matrix effects (15). Useful constitutively active promoters include but at not limited to cytomegalovirus (CMV) early enhancer/promoter, SV40 promoter, UBC promoter, PGK promoter, human β-actin (hACTB), human elongation factor-1α (hEF-1α), and cytomegalovirus early enhancer/chicken β-actin (CAG) promoters.

As mentioned herein, present invention also provides a method for detecting and operationally quantitating the activity of neutralizing antibodies against recombinant AAV vectors in a test sample, said method comprising the steps of:
(i) providing a test sample(s), thought to contain antibodies against a recombinant AAV vector together with control sample(s) without anti-AAV antibodies and a positive control sample(s) known to contain neutralizing antibodies against the AAV vector
(ii) contacting said test sample(s) with either cell-free recombinant AAV vector or the virus-producing packaging cells according to the present invention, said recombinant AAV vector expresses a sequence tag within the genome of the AAV serotype or transgene promoter construct, such as for example the Gal4 DNA binding domain,
(iii) contacting said test sample(s) with either the cell-free recombinant AAV vector or the virus-producing packaging cells according to the present invention, with the reporter cells, said cells expressing a reporter-gene-promoter construct, for example firefly luciferase under the control of a tandem-repeat of the Gal4 UAS that will respond specifically to the tag sequence within the genome of the recombinant AAV vector or transgene construct produced by the packaging cells.
(iv) Optionally, and in a preferred embodiment, to provide for normalization of the assay, the reporter cell line according to the present invention further contains a construct for the constitutive production of a reporter-gene that is different from that used in the AAV-responsive reporter gene construct. For example, the constitutive production may be of a second luciferase, such as e.g., Renilla luciferase, Metridia luciferase or a novel luciferase such as e.g. that described EP application 21170068.7. In a preferred embodiment the reporter-gene cell line according to the present invention, is seeded in 96, 384, or 1536 well assay plates.
(v) determining the activity of the first reporter protein in said cell line using a suitable substrate such as e.g. the commercially available substrate Dual-Glo or any commercially available luciferase substrate,
(vi) determining the activity of the second reporter protein in said cell line after the tag-responsive reporter gene luciferase is measured in the same sample using for example the Stop & Glo reagent from the Dual-Glo system or any commercially available luciferase substrate that efficiently inhibits the activity of the first reporter protein for example firefly luciferase (Lallemand C. et al J Immunol Res. 390:1-19, 2017, incorporated herein by reference in its entirety).
(vii) The activity of the first luciferase normalized relative to the activity of the second luciferase is described in US 2011/0189658, incorporated herein by reference in its entirety.
(viii) determining the activity of the first tag-responsive reporter protein in the reporter cells of the first cell sample normalized or not relative to the activity of the second luciferase is described in US 2011/0189658, incorporated herein by reference in its entirety
(ix) and determining the activity of the first tag-responsive reporter protein in the cells of the second control cell sample, normalized or not relative to the activity of the second luciferase is described in US 2011/0189658, incorporated herein by reference in its entirety.
(x) provide the ratio between the reporter activity in first and a second control cell sample, where a ratio (first/second) lower than one is indicative for the presence of antibodies against the recombinant AAV vector in said sample.

In a further aspect of the present invention relates to a method for high throughput screening of antibodies against recombinant AAV vectors said method comprising the steps of
(i) providing a test sample(s), thought to contain antibodies against the recombinant AAV vector together with control sample(s) without anti-AAV antibodies and in a preferred embodiment an anti-AAV standard sample(s)
(ii) contacting said test sample(s) and control sample(s) with the challenge virus preparation or the virus-producing packaging cells according to the present invention, said cells for the production of the recombinant AAV vector, to which the antibody response is to be determined, said recombinant AAV vector contains a tag sequence within the virus genome or the transgene construct.
(iii) Contacting said test sample(s), control sample(s) and challenge virus/virus-producing packaging cells with a reporter-gene cell line according to the present invention, said reporter cell line containing a first heterologous polynucleotide comprising a heterologous cis-acting regulatory sequence, that responds to treatment of the cell line with the tag sequence present within the genome of the AAV serotype or the transgene-promoter construct of the
   recombinant AAV vector, to which antibodies present in the test sample(s) are to be detected or detected and quantified.
(iv) wherein said promoter of the reporter cell line is operably linked to an open reading frame encoding a first reporter protein comprising for example firefly luciferase, Renilla luciferase, Metridia luciferase or a novel luciferase such as that described EP application 21170068.7. In a preferred embodiment the cell line according to the present invention, is seeded in 96, 384, or 1536 well assay plates.
(v) In a preferred embodiment, to provide for a normalization of the assay, the reporter-cell line according to the present invention further has a construct for the constitutive production of a luciferase that is different from that used in the AAV-responsive reporter gene construct. For example, the constitutive production may be of a second luciferase, e.g., Renilla luciferase, firefly luciferase, Metridia luciferase or a novel luciferase such as that described in EP application 21170068.7 determining the activity of the first reporter protein in said cell line using a suitable substrate such as the commercially available Dual-Glo luciferase reagent from the Dual-Glo system , or any commercially suitable luciferase substrate.
(vi) determining the activity of the second reporter protein in said cell line after the tag-responsive reporter gene luciferase is measured in the same sample using for example the Stop & Glo reagent from the Dual-Glo system (Promega, Madison, WI) that efficiently inhibits the activity of the first reporter protein. The activity of the first luciferase normalized relative to the activity of the second luciferase is described in US 2011/0189658 incorporated herein by reference in its entirety.
(vii) determining the activity of the first tag-responsive reporter protein in the reporter cells of the first cell sample normalized or not relative to the activity of the second luciferase is described in US 2011/0189658, incorporated herein by reference in its entirety.
(viii) and determining the activity of the first tag-responsive reporter protein in the cells of the second control cell sample, normalized or not relative to the activity of the second luciferase is described in US 2011/0189658, incorporated herein by reference in its entirety.
(ix) provide the ratio between the reporter activity in first and a second control cell sample, where a ratio (first/second) lower than one is indicative for the presence of antibodies against the recombinant AAV vector in said sample.

Consequently, the present invention provides packaging cell lines transfected with genes encoding the genome of a specific recombinant AAV vector expressing a naturally occurring capsid, hybrid capsid, or chimeric capsid and encoding a specific transgene/promoter construct labelled with a specific tag sequence, and a reporter cell line that responds specifically to the tag sequence encoded by the specific recombinant AAV vector. If a common tag sequence is used to label each specific recombinant AAV vector, then a single reporter cell line can be used to detect all the different recombinant AAV vectors labelled with the same tag sequence. If, however, a different tag sequence is used to label an individual recombinant AAV vector then either a different reporter cell line that responds specifically to each individual specific tag sequence, or a reporter cell line that contains reporter-gene(s) constructs that respond to each individual tag is required. The use thereof of the present invention in various contexts allowing for the detection of the activity of neutralizing antibodies with a higher sensitivity, such that the EC₅₀ of the doseresponse curve of a sample containing neutralizing antibodies directed against a particular AAV serotype or recombinant AAV vector determined according to the present invention is at least decreased in comparison with prior art methods. The decrease may be about 2-fold, such as e.g. about 3-fold, such as e.g. about 4-fold, such as e.g. about 5-fold, such as e.g. 6-fold, such as e.g. 7-fold, such as e.g. about 8-fold, such as e.g. 9-fold, such as e.g. 10-fold, such as e.g. 50-fold, such as about 100-fold, or such as about 1000-fold.

In one aspect, the present invention relates to cells or cell lines and methods in relation thereto enabling higher specificity. The specificity may be at least about 75%, such as e.g. at least about 80%, such as e.g. at least about 85%, such as e.g. at least about 90%, such as e.g. at least about 95%, such as e.g. at least about 97.5%, such as e.g. at least about 98%, such as e.g. at least about 99%, such as e.g. at least about 99.5%.

In one aspect, the present invention provides for an increased sensitivity.

In another aspect, the present invention provides for an increased specificity.

In yet a further aspect, the present invention provides for both an increased sensitivity as well as for an increased specificity.

### Brief description of the drawings

Fig. 1 illustrates the molecular constructs used to establish **Component 1: The Packaging Cell Line of Example 1.** The human lung adenocarcinoma cell line A549 was stably transfected with the following constructs; the transgene of choice and a gal4-VP16 tag sequence separated by a sequence encoding a 2A self-cleaving peptide, F2A in the example shown, under the control of a suitable promoter, a CMV constitutive promoter in the example shown, and a polyadenylation site, of SV40 in the example shown, contained within the ITRs of a given AAV serotype, AAV2 in the example shown.

The *rep* and *cap* genes are expressed on different plasm ids under the control of constitutive promoters.

The *E1* gene is expressed under the control of an inducible promoter, the rapamycin-inducible promoter consisting of a 12-fold tandem repeat of the ZFHD1 DNA-binding domain fused to the FL506 binding protein (KFBP), in the example shown.

The cells have also been stably transfected with the genes encoding E2A, E4, and VA RNA under the control of constitutive promoters

Fig. 2 illustrates the molecular constructs used to establish **Component 2: The Reporter Cell Line of Example 1.** The human embryonic kidney cell line HEK293 was stably transfected with a chimeric promoter consisting of a 5-fold tandem repeat of the Gal4 upstream activation sequence (UAS) and a minimal promoter regulating expression of the firefly luciferase (FL) gene together with a polyadenylation site, from SV40 in the example shown.

Fig. 3 illustrates the release of the recombinant AAV vector from the packaging cell and uptake into the reporter-cell line when the two components are placed in contact with each another.

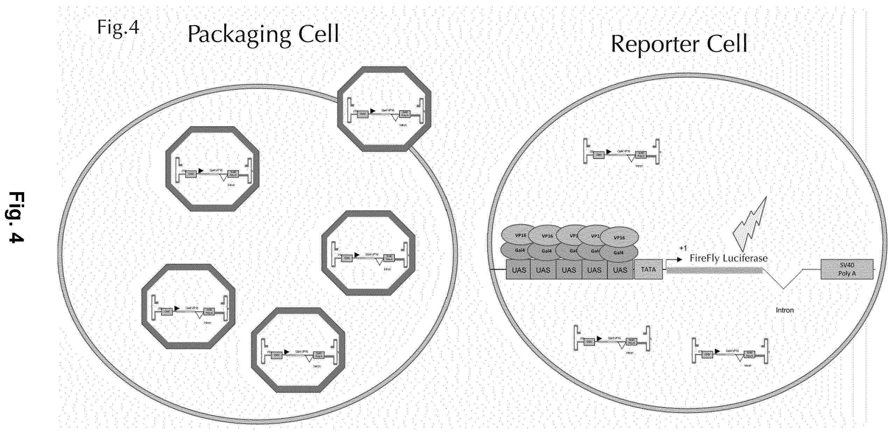

Fig. 4 illustrates the expression of the genome of the recombinant AAV vector and binding of the Gal 4 DNA binding domain-VP6 hybrid protein to the 5-fold tandem repeat of the Gal4 upstream activation sequence (UAS) and activation of the firefly luciferase (FL) gene.

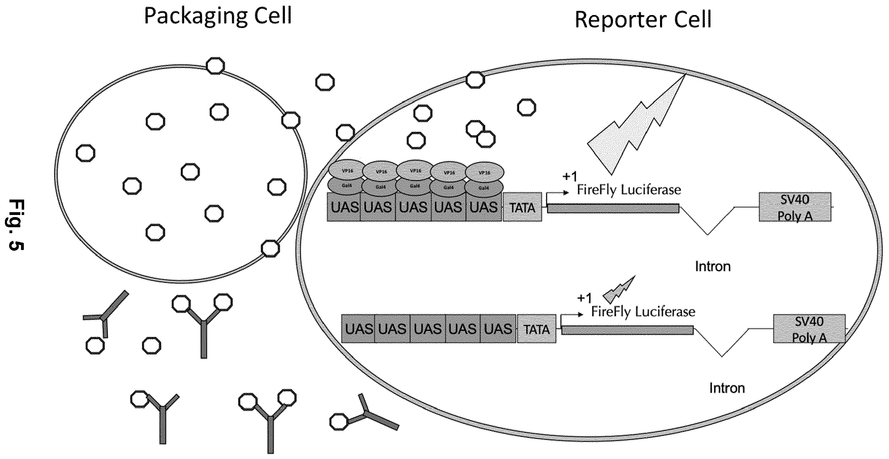

Fig. 5 illustrates the neutralization of AAV viral particles by anti-AAV antibodies present in a sample as they immerge from the packaging cell line (Component 1) preventing them from entering the reporter-cell line (Component 2) and activating the Gal-4-UAS regulated firefly luciferase reporter-gene. This results in a lower firefly luciferase signal in the presence of neutralizing anti-AAV antibodies.

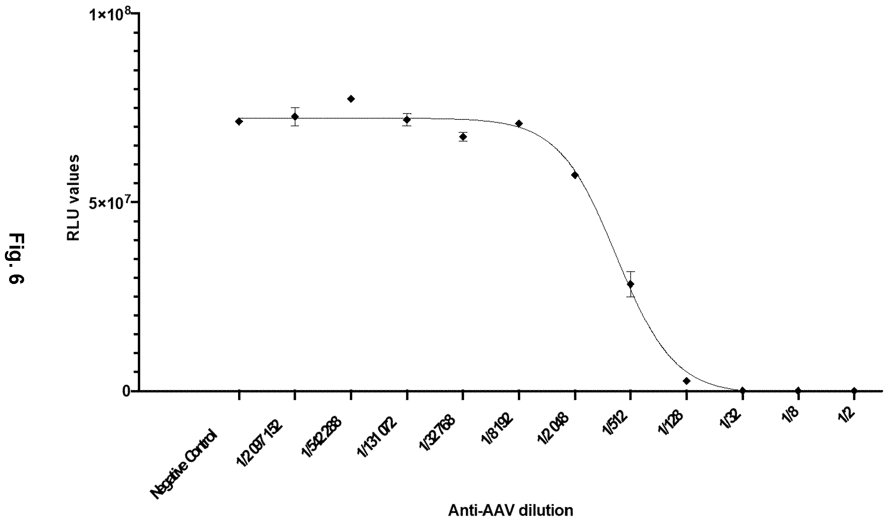

Fig. 6 illustrates the quantification of the neutralizing activity of a serum sample following contacting the packaging cell line expressing a recombinant AAV2 vector with the sample to be tested or not for 30 minutes at 20°C prior to incubation of the packaging cell line and the sample with the reporter-cells at 37°C for 18 hours prior to quantification of FL reporter-gene activity using a commercially available substrate (Bright-Glo, Promega, Madison, WI) and quantification of light emission in a luminometer (Glo-Max, Promega, Madison, WI).

Fig. 7 illustrates the potentiation of Gal4-UAS regulated FL expression following transient transfection of the reporter-cell line (Component 2) with the gene encoding the immediate early enhancer protein E4orf6 and incubation of the reporter cells with packaging cells (Component 1) containing a cap gene encoding the AAV2 cap proteins.

Fig. 8 illustrates the potentiation of Gal4-UAS regulated FL expression following transient transfection of the reporter-cell line (Component 2) with the gene encoding the immediate early enhancer protein E4orf6 and incubation of the reporter cells with packaging cells (Component 1) containing a cap gene encoding the AAV5 cap proteins.

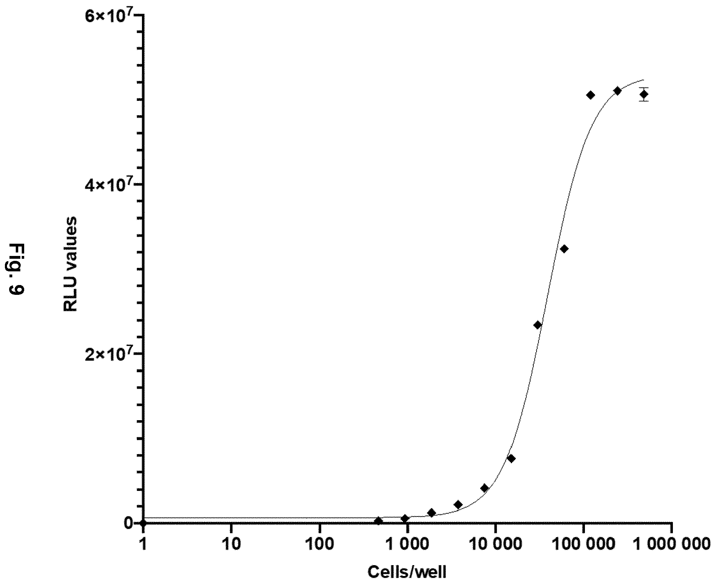

Fig. 9 illustrates the relationship between the number of reporter cells (Component 2) seeded per well of a microtiter plate and Gal4-UAS regulated FL expression in the presence of the packaging cells (Component 1) containing a cap gene encoding the AAV2 cap proteins. A total of 15,000 reporter cells/well in the presence of 7,500 packaging cells was found to give optimal FL expression.

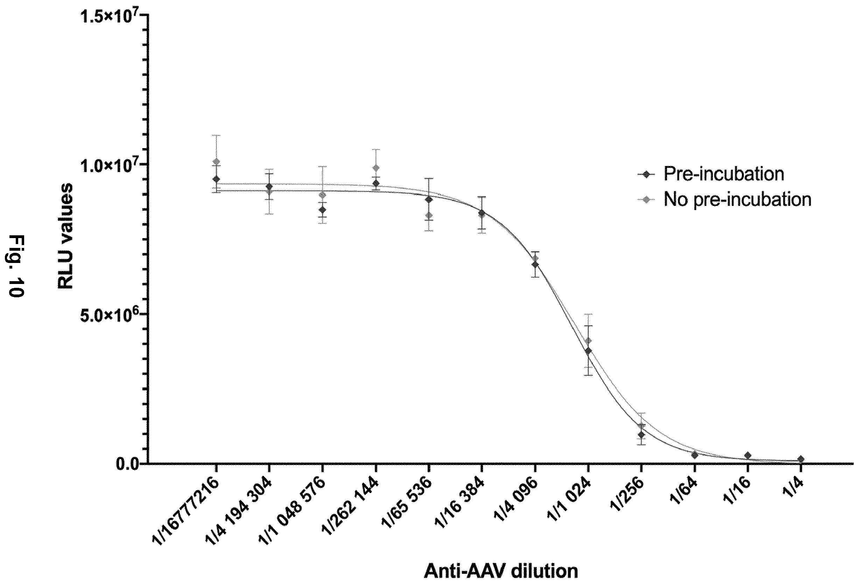

Fig. 10 illustrates the effect of pre-incubating or not the samples to be tested for the presence of anti-AAV2 neutralizing antibodies with the packaging cells (Component 1) expressing the gene encoding the AAV2 Cap proteins for 30 minutes at room temperature prior to incubation with the reporter-cells (Component 2) for 18 hours at 37°C.

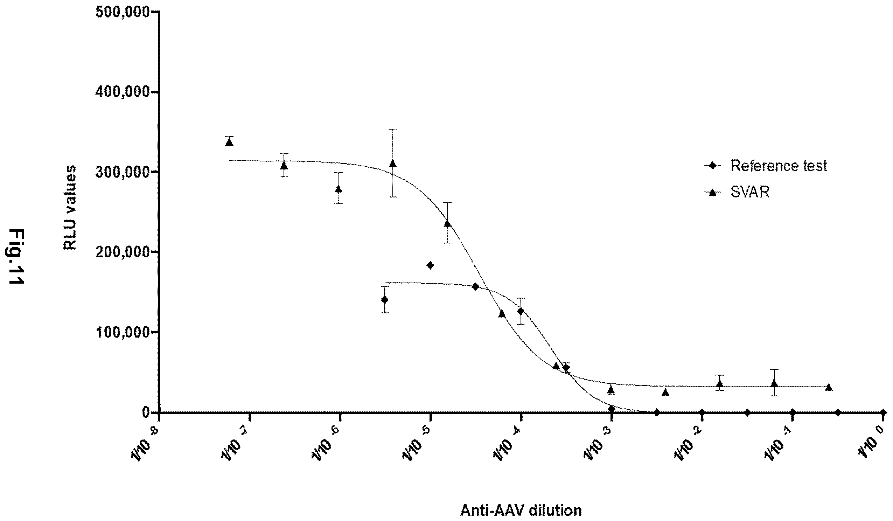

Fig. 11 illustrates a comparison of the titration of a sample tested for the presence of anti-AAV2 neutralizing antibodies using either the invention described herein or the transduction inhibition reference method.

### Detailed description of the invention

In describing the embodiments of the invention specific terminology will be resorted to for the sake of clarity. However, the invention is not intended to be limited to the specific terms so selected, and it is understood that each specific term includes all technical equivalents which operate in a similar manner to accomplish a similar purpose.

The present invention was made in view of the prior art described above, and the object of the present invention is to provide a novel system for the detection and quantification of neutralizing antibodies, directed against recombinant adeno-associated virus (AAV) vectors, with improved sensitivity and specificity. To achieve this goal the present invention provides i.a. for a packing cell line **(Component 1)** for the production of the recombinant AAV vector, to which an antibody response may be determined, that comprises a tag sequence within the virus genome or the transgene promoter construct contained within the AAV ITRs.

Present invention also relates to a reporter-gene cell line incorporating a reporter-gene promoter construct that responds specifically to the tag sequence of Component 1 **(Component** 2) and a method of using the same for detecting and optimally quantitating anti-AAV neutralizing antibodies in a test sample.

The invention further relates to a method for high-throughput screening for detecting and optimally monitoring the immune response to recombinant AAV vectors with optimal sensitivity, signal strength, and biological fidelity. Furthermore, present invention also relates to use of Component 1 and/or Component 2 in diagnostics or a diagnostic methods, such as e.g. for the detection and quantification of neutralizing antibodies (NAbs), directed against recombinant adeno-associated virus (AAV) vectors.

### Component 1: Packaging Cell Line

As mentioned herein, the present invention relates to an AAV packing cell line which may comprise the constituent components required for the expression of the recombinant AAV vector are supplied by transient transfection or preferably have been partially or completely integrated into the genome of the chosen cell line. The chosen cell line may be referred to as a host cell or host cell line.

Thus, the present invention relates to a cell or cell line **(Component 1**), the cell comprising at least one or more of;
(i). the AAV genome or the transgene-promoter construct comprising one or more tag sequences contained within the AAV ITRs,
(ii). the *cap* gene of the recombinant AAV vector, encoding either a naturally occurring Cap, hybrid, Cap or chimeric Cap operationally linked to a constitutive promoter,
(iii). the AAV *rep* gene, operationally linked to an inducible promoter,
(iv). the AAV *E2A, E4, and VA* genes, each operationally linked to different individual natural or inducible promoters,
(v). the AAV *E1A* gene, operationally linked to an inducible promoter.

In one aspect, the cell comprises several or all of (i), (ii), (iii), (iv), and (v). In a particular aspect, the cell comprises all of (i), (ii), (iii), (iv), and (v).

In one aspect, the inducible promotors in (iii), (iv), and (v) are different from each other and may be selected from the Tet-on/Tet-off system, the cumateinducible repressor CymR system, the flavonoid phloretin regulated TtgR repressor system, and the vanillic acid regulated VanR repressor/KRAP system.

It is understood that in order to increase sensitivity, the host cell line may be stably transfected with either the cell surface AAV receptor alone (9), or the endosomal AAV receptor alone (10), or both the cell surface receptor and the endosomal AAV receptors.

The choice of host cell line is determined by its safety profile for the analysis of biological samples and the ability of the cells to tolerate the toxicity of the AAV Rep protein following regulated expression of the rep gene and E4orf6 (11). In one aspect, the host cell me be a metazoan cell. In another aspect, the host cell lines may include, but are not limited to, the human lung adenocarcinoma cell line A549 (ATCC catalogue # CCL-185) or the human embryonic kidney cell line HEK293T (ATCC catalogue # ACS-4500).

The chosen host cell line may be transfected with the transgene of choice under the control of either a constitutive promoter, such as e.g. the CMV promoter, or an appropriate tissue-specific promoter regulating expression of the transgene, chosen as a function of the desired tissue-specific expression of the transgene, and a suitable polyadenylation site such as e.g. that from SV40 or e.g. an alternative polyadenylation site contained within the ITRs of AAV2 (Figure 1). In another aspect, the constitutive promotor may be e.g. SV40, UBC, EF1A, PGK, and the CAGG promoters. In yet a further aspect, the tissue specific promotor may be e.g. human retina-specific promoters include the red opsin 2.1 & 1.7 promoters, the green opsin 2.1 & 1.7 promoters, the rhodopsin kinase 2 (hGPK1) promoter, the cone arrestin hCAR promoter, the vitelliform macular dystrophy (VMD2)promoter. Common muscle-specific promoters include the creatine kinase (CK), and myosin heavy chain (MyHC)promoters, or neuron specific promoter such as the synapsin promoter.

The host cells may further be co-transfected with the *cap* gene under the control of a constitutive promoter such as e.g. the CMV immediate early promoter (Figure 1). The cells are co-transfected with the *cap* and *rep* gene on two independently regulated since expression of the *cap* and *rep* genes on separate plasm ids has been reported to increase AAV vector production (12).

The gene encoding E1 and the E1A and E1B ORFs, is not expressed in A549 cells in contrast to HEK293T cells, and thus A549 cells were transfected with the *E1* gene under the control of a suitable inducible promoter to prevent the E1a protein activating the *Rep, E2,* and *E4* genes. Thus, as is illustrated in Example 1 and in one aspect of the invention the *E1* gene is expressed under the control of a rapamycin-inducible promoter consisting of a 12-fold tandem repeat of the DNA binding domain ZFHD1 fused to the FK506 binding protein (FKBP). The FKBP-rapamycin-associated protein (FRAP) is fused to the VP16 trans-activator from HSV-1 such that addition of rapamycin will induce the formation of hetero-dimers between FKBP and FRAP leading to a tightly controlled expression of the gene encoding the E1 early protein.

Controlled expression of Rep78/68 has also been shown to increase the level of AAV vector production (9). Although transfection of HEK293 cells with *E4 orf6* alone has been reported to be sufficient for adenovirus-free production of a recombinant AAV vector (14) the early helper proteins E2A, E4, and VA RNA have been reported to be required for efficient production of recombinant AAV (15). Thus, the packaging cell line may in one instance be co-transfected with expression vectors expressing E2A, E4, and VA RNA.

### Component 2: Reporter Cell Line.

In one aspect, the invention relates to a reporter-gene cell line (also referred to herein as Component 2) has been developed that may respond specifically to the tag sequence contained within the AAV genome or transgene construct of Component 1, such that contacting the reporter cells with the recombinant AAV vector challenge virus or the packaging cells producing the required recombinant AAV vector to which neutralizing antibodies are to be quantified will result in activation of the reporter-gene expressed by the reporter-cell line. In Example 1 the packaging cell line expresses a transgene-promoter construct containing a tag sequence encoding the Gal4 DNA binding domain, that will bind specifically to the Gal4 upstream activation sequence (UAS) regulating expression of the firefly luciferase (FL) gene. Specifically, in Example 1 the Reporter Cells contain a 5-fold tandem repeat of the Gal4 UAS, regulating expression of the FL reporter-gene in a HEK293 cell background (Figure 1).

### Interaction of the packaging cell line and the reporter cells

In various embodiments, the present invention relates to a method, the method comprising at least one of:
(a). contacting the virus produced by the packaging cells (Component 1) according to the present invention with the sample(s) to be tested for various times and at various temperatures including either about +4°C, about 20°C, or about 37°C prior to incubation of the virus preparation and the sample(s) with the reporter-cells (Component 2) at about 37°C for various times preferably from about 6 to about 18 hours or more prior to quantification of FL reporter-gene activity using a suitable substrate such as the commercially available substrate Bright-Glo (Promega, Madison, WI).
(b). contacting the virus-producing packaging cells (Component 1) with the sample(s) to be tested for various times at various temperatures including either about +4°C, about 20°C, or about 37°C prior to incubation of the virus producing packaging cells and the sample(s) with the reporter-cells (Component 2) at about 37°C for various times preferably for about 6 to about 18 hours or more prior to quantification of FL reporter-gene activity.
(c). and in a preferred embodiment contacting the virus-producing packaging cells (Component 1) with the sample(s) to be tested and the reporter-cells (Component 2) and incubating directly at about 37°C for various times preferably for about 6 to about 18 hours or more prior to quantification of FL reporter-gene activity using a suitable substrate such as the commercially available substrate Bright-Glo (Promega, Madison, WI).
(c). freezing the virus-producing packaging cells (Component 1) together with the reporter cells (Component 2) at the appropriate cell concentrations in a suitable cryo-protective medium, thawing the cells, contacting the packaging cells/reporter-gene cells with the sample(s) to be tested and incubating the sample(s), packaging cells-reporter-cells at about 37°C for various times preferably for about 18 hours or more prior to quantification of FL reporter-gene activity using a suitable substrate such as the commercially available substrate Bright-Glo (Promega, Madison, WI).
(d). in a preferred embodiment freezing the virus-producing packaging cells (Component 1) and the reporter cells (Component 2) separately at the appropriate cell concentrations in a suitable cryo-protective medium, thawing the cells, contacting the packaging cell line with the sample(s) to be tested for various times and at various temperatures including either about +4°C, about 20°C, or about 37°C prior to incubation of the packaging cells and the sample(s) with the reporter-cells at about 37°C for various times preferably for about 18 hours or more prior to quantification of FL reporter-gene activity using a suitable substrate such as the commercially available substrate Bright-Glo (Promega, Madison, WI).

In a preferred embodiment about 7,500 packaging cells (Component 1) are mixed with about 15,000 reporter cells (Component 2) as shown in Fig. 9, without pre-incubation of the samples with the packaging cells (Component 1) prior to contact with the reporter cells (Component 2) and incubation at 37°C for approximately 18 hours (Figure 10).

In one aspect, the AAV preparation or virus-producing packaging cells may interact with the reporter-gene cells resulting in virus uptake, internalization, and AAV-serotype specific activation of the FL reporter-gene. The initial interaction of the virus preparation or virus-producing packaging cells with the reporter cells is most probably mediated by the release of virus & interaction of the capsid with carbohydrates on the cell surface in a serotype-specific interaction. Thus, AAV serotypes 2, 3, and 6 are known to bind to heparin sulfate proteoglycan, while AAV1, 4, 5, and 6 bind sialic acid and AAV9 binds to galactose (8). The AAV serotypes then interact with either the AAV receptor required for cellular internalization (9) and/or the novel G protein-coupled receptor like protein GPR108, localized in the *trans-Golgi* (10). In one embodiment, the reporter gene encodes an enzyme or any other means enabling detection in a suitable manner. In a preferred embodiment, the reporter is a luciferase, such as firefly luciferase or Renilla luciferase, Metridia luciferase or a novel luciferase present in solution as soluble active monomers, or as fusion proteins with other proteins including luciferases or fluorescent proteins such as green fluorescent proteins (GFPs) enhanced green fluorescent protein (EGFP), red fluorescent proteins (RFPs), yellow fluorescent protein (YFP), blue fluorescent protein (BFP) and variants there of displaying a different excitation/emission spectra, horseradish peroxidase (HRP), or various conjugates thereof, secreted human placental alkaline phosphatase (SEAP), or various conjugates thereof, chloramphenicol acetyltransferase (CAT), or various linker proteins, or attached to solid surfaces such as particles, beads, assay-plates or tubes.

In another embodiment, the reporter gene encodes a fluorescent protein. Useful fluorescent protein includes green fluorescent protein (GFP) and related fluorescent proteins, e.g. enhanced green fluorescent protein (EGFP), red fluorescent protein (RFP), yellow fluorescent protein (YFP), blue fluorescent protein (BFP) and variants thereof displaying a different excitation/emission spectra.

In a further embodiment the reporter-cells are co-transfected with a first reporter-gene such as the firefly luciferase (FL) reporter-gene under the control of a chimeric promoter that responds specifically to the tag sequence present within the recombinant AAV encoded transgene construct (resulting from Component 1) and a second reporter-gene such as the Renilla luciferase (RL) under the control of a constitutive promoter that allows the tag sequence activated FL activity to be normalized with respect to the constitutive level of expression of RL activity rendering the assay independent of cell number and providing a means for compensating for inter-sample variation in cell densities due to loss of cells or variation in number of cells seeded. The ability to normalize AAV-activated FL activity relative to the constitutive express of RL activity also provides a means to compensate for serum matrix effects (16). Useful constitutively active promoters include but at not limited to cytomegalovirus (CMV) early enhancer/promoter, SV40 promoter, UBC promoter, PGK promoter, human β-actin (hACTB), human elongation factor-1α (hEF-1α), Thymidine Kinase (TK) promoter and cytomegalovirus early enhancer/chicken β-actin (CAG) promoters.

The present inventors provide a novel system for the detection and quantification of neutralizing antibodies directed against recombinant AAV vectors with improved sensitivity, precision, and specificity.

To solve the technical problem as outlined above, a two-component system is described comprising a packing cell line **(Component 1)** for the production of the recombinant AAV vector, to which the antibody response is to be determined, that contains a tag sequence within the virus genome or the transgene promoter construct contained within the AAV ITRs and a reporter-gene cell line incorporating a reporter-gene that responds specifically to the tag sequence **(Component 2)** and a method of using the same for detecting and optimally quantitating anti-AAV neutralizing antibodies in a test sample(s). The invention further relates to a method for high-throughput screening for detecting and optimally monitoring the immune response to treatment with recombinant AAV vectors, with optimal sensitivity, signal strength, and biological fidelity.

**Component** 1: **Packaging Cell Line.** A stable AAV packing cell line may be established in which the constituent components required for the expression of the recombinant AAV vector are supplied by transient transfection or in a preferred embodiment of the preset invention have been integrated into the genome of the chosen cell line.

As a non-limiting example, a stable cell line for the production of a specific recombinant AAV vector may be established by transfecting the human lung adenocarcinoma cell line A549 (ATCC catalogue # CCL-185) with the transgene of choice under the control of either a constitutive promoter or an appropriate tissue-specific promoter chosen as a function of the intended use, and a polyadenylation site contained within the ITRs of AAV2 (as illustrated in Figure 1).

To construct an expression vector for the transgene-promoter construct that will confer the highest level of expression possible, various types of variants of the promoter are designed *in silico* to reduce both the size of the promoter and to increase transcription of the transgene. The codon-optimized transgene-promoter constructs containing a tag sequence such as e.g. the Gal4 DNA binding domain of Example 1 are then synthesized *in vitro* and used to construct a series of chimeric transgene-promoter sequences and either a synthetic polyadenylation site, the SV40 polyadenylation site or the human growth hormone polyadenylation site, chosen as a function of its efficacy, contained within the ITRs of AAV2. Transgene expression can be increased by modifying the ITRs to generate self-complementary intermediates such that the transgene is expressed without the need for second-strand DNA synthesis (13). These constructs are then tested for their ability to drive transcription of the transgene in a series of transient transfection experiments in a packaging cell line established for example in A549 cells, that contains all the early helper proteins required to replicate a transgene encoded by an AAV vector but lacks the transgene-promoter construct with the tag sequence contained within the ITRs of AAV2. Expression levels are monitored by Western blot using the appropriate detection antibodies and by the level of activation of the firefly luciferase (FL) reporter-gene under the control of a chimeric transgene responsive promoter. Results are normalized with respect to expression of Renilla luciferase (RL) following co-transfection of the cells with the RL reporter gene under the control of a constitutive promoter.

If the normalized level of expression of the transgene monitored by Western blot is estimated to be optimal relative to that of the standard ITR and the normalized increase in FL expression is estimated to be sufficient determined using the Reporter Cell Line such as that of Example 1 that expresses a tandem repeat of the Gal4 UAS regulating expression of the firefly luciferase reporter gene that responds specifically the Gal4 DNA binding domain tag contained within the AAV genome or recombinant AAV vector, then the construct(s) are used to establish a Packaging cell Line either by transient transfection or in a preferred embodiment by the establishment of a stable packaging cell line in which the required components are stably integrated into the genome of a host cell line such as A549 cells.

The packaging cell line may be transfected with an expression vector for the cap gene under the control of a constitutive promoter. Thus, in Example 1 the cap gene is expressed under the control of the CMV immediate early promoter and is used to establish a Packaging Cell Line following transient transfection with the constituent components or in a preferred embodiment by the establishment of a stable packaging cell line in which the required components are stably integrated into the genome of a host cell line such as for example A549 cells.

To ensure optimal regulated expression of the *rep* gene and early genes required to express the transgene in Example 1 the gene encoding E1 was expressed under the control of a rapamycin-inducible promoter consisting of a tandem repeat of a variant of the DNA binding domain ZFHD1 fused to the FK506 binding protein (FKBP) designed *in silico.* Variants of the FKBP-rapamycin-associated protein (FRAP) were fused to the VP16 trans-activator from HSV-1 also designed *in silico* such that addition of rapamycin induces the formation of hetero-dimers between FKBP and FRAP leading to a tightly controlled expression of the gene encoding the E1 early helper protein.

The constructs were then synthesized *in vitro* and tested for their ability to regulate expression of the E1 protein in a series of transient transfection experiments in the A549 packaging cell line. Proteins encoded by the *E1A* gene initiate AAV replication by increasing *rep* expression by transactivating the p5 and p19 promoters. To ensure controlled expression of the Rep78/68 proteins that have been shown to increase the level of AAV vector production (14), the rep gene were also placed under the control of the ZFHD1 promoter. Tandem repeats of the DNA binding domain ZFHD1 fused to the FK506 binding protein (FKBP) and variants of the FKBP-rapamycin-associated protein (FRAP) fused to the VP16 trans-activator were designed *in silico,* synthetized *in vitro* and tested in transient transfection experiments in A549 cells such that addition of rapamycin induces the formation of hetero-dimers between FKBP and FRAP leading to a tightly controlled expression of the gene encoding the E1 early enhancer protein.

In Example 1 the early enhancer proteins E2A, E4, and VA RNA that are required for efficient production of recombinant AAV were expressed as a single polycistronic sequence incorporating self-cleaving 2A peptides, to ensure that the 3 individual proteins are expressed at the required level and with a constant stoichiometry, under the control of E1a in A549 cells expressing E1 under the control of the ZFHD1 promoter. The functionality of the construct(s) was monitored by the efficiency of production of the normalized level of expression of the encapsulated transgene determined using the Reporter Cells of the present invention.

In a preferred embodiment of the preset invention the constituent components of the Packaging Cell Line have been integrated into the genome of the chosen cell line as described in Example 1. Human A549 cells were co-transfected with the optimal transgene-promoter construct developed. The cells were also co-transfected with the *rep* gene and *E1A* gene both under the control of rapamycin regulated ZFHD1 promoters as well as the *E2A, E*4 and *VA* genes, the expression of which is regulated by E1A expression. Human A549 cells were transfected with the above constructs using an integrase/transposon system that insures integration of the transgenes in a transcriptionally active region of chromatin. Individual stable clones were isolated and characterized for rapamycin inducibility following transient co-transfection with a vector expressing the firefly luciferase (FL) gene under the control of a 12-fold tandem repeat of the ZFHD1 promoter and by Western blot for expression levels of E1A, Rep and E2/E4. Individual clones were also tested for the expression levels of whole encapsulated competent recombinant AAV vector determined by qPCR. Quantification of AAV vector genomes was based on the use of an AAV2 ITR-specific target sequence that allows a direct comparison of results between laboratories but is dependent upon the use of a linear or circular ITA standard and an extended denaturation cycle due to the extensive secondary hairpin structure of the AAV2 ITRs (15).

The stability of the selected clone was tested at regular intervals for 20 passages to determine the stability of the transgenes in human A549 cells. The clone selected exhibited no loss of the level of whole encapsulated competent recombinant AAV vector monitored by the level of activation of the transgene responsive Reporter Cell Line. The clone also exhibited stable growth characteristics including both doubling time and the maximum cell density attained under standardized growth conditions in culture medium containing the required selective agents.

### Definitions

### Vector

The term "vector" or vector construct" refers to a DNA molecule used as a vehicle to transfer recombinant genetic material into a host cell. The four major types of vectors are plasmids, bacteriophages and other viruses, cosmids, and artificial chromosomes. The vector itself is generally a DNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences. The choice of vector employed in embodiments of the present invention depends on the specific application of the vector encoding the polypeptides or polynucleotide.

### Transgene

A transgene is a segment of DNA encoding an open reading frame (ORF), initiation and termination codons and that is transferred often from one organism to another using a suitable vector.

### Operatively linked

The term "operatively linked" refers to the connection of elements being a part of a functional unit such as a gene or an open reading frame. Accordingly, by operatively linking a promoter to a nucleic acid sequence encoding a polypeptide (an open reading frame, ORF) the two elements become part of the functional unit - a gene. The linking of the expression control sequence (promoter) to the nucleic acid sequence enables the transcription of the nucleic acid sequence directed by the promoter. By operatively linking two heterologous nucleic acid sequences encoding a polypeptide the sequences become part of the functional unit - an open reading frame encoding a fusion protein comprising the amino acid sequences encoding by the heterologous nucleic acid sequences. By operatively linking two amino acids sequences, the sequences become part of the same functional unit - a polypeptide. Operatively linking two heterologous amino acid sequences generates a hybrid (fusion) polypeptide.

### Minimal constitutive promoter

The promoter directing the expression of a reporter gene is typically minimally constitutively active in the mammalian host cell used to establish the reporter cell line of the present invention and alone does not respond to treatment of cells with a pharmacologically active molecule. Useful constitutively active promoters include but at not limited to cytomegalovirus (CMV) early enhancer/promoter, SV40 promoter, UBC promoter, PGK promoter, human β-actin (hACTB), human elongation factor-1α (hEF-1α), Thymidine Kinase (TK) promoter and cytomegalovirus early enhancer/chicken β-actin (CAG) promoters.

### Inducible promoter

An inducible promoter is a DNA sequence from which transcription of mRNA takes place following a reversible changes in the presence or abundance or conformation of a regulatory factor or protein(s) in a cell, which enable activating transcription factors to recruit RNA polymerase II. According to present invention, an inducible promoter may be any promoter responding to or activated (induced) by chemical agents, temperature, and light are all examples of factors that can lead to the induction of a promoter.

Chemically regulated promoters are among the most common inducible promoters. The positive inducible tetracycline ON (Tet-On) system, a versatile tool developed for use in prokaryotes and eukaryotes, works via direct activation. In this system, the activator rtTA (reverse tetracycline-controlled transactivator) is normally inactive and cannot bind the tetracycline response elements (TRE) in a promoter. Tetracycline and its derivatives serve as inducing agents to allow promoter activation.

One of the most commonly used prokaryotic promoters is the negative inducible pLac promoter. This promoter requires removal of the lac repressor (lacl protein) for transcription to be activated. In the presence of lactose or lactose analog IPTG, the lac repressor undergoes a conformational change that removes it from lacO sites within the promoter and ceases repression of the target gene. A simplified lac inducible system is found in many bacterial expression vectors.

Negative inducible promoter pBad is another popular prokaryotic promoter often used for bacterial protein purification. When arabinose is absent, regulatory protein AraC binds O and I1 sites upstream of pBad, blocking transcription. The addition of arabinose causes AraC to bind I1 and I2 sites, allowing transcription to begin. In addition to arabinose, cAMP complexed with cAMP activator protein (CAP) can also stimulate AraC binding to I1 and I2 sites. Supplementing cell growth media with glucose decreases cAMP and represses pBad, decreasing promoter leakiness.

Temperature sensitive expression systems are typically less leaky than chemically induced promoters; they show near-zero expression at regular temperatures but can be induced by heat or cold exposure. Examples include, but are not limited to the heat shock-inducible Hsp70 or Hsp90-derived promoters, in which a gene of choice is only expressed following exposure to a brief heat shock. In the case of Hsp70, the heat shock releases heat shock factor 1 (HSF-1), which subsequently binds to heat shock elements in the promoter, thereby activating transcription. Other non-limiting examples are heat shock-inducible Cre and Cas9 for genome engineering in species like e.g. *C. elegans* and *Drosophila.*

Light is another way to activate gene expression, and two-component systems used in synthetic biology use light to regulate transcription. Red flame plasmid pDawn contains the blue-light sensing protein YFI. When light is absent, YFI phosphorylates FixJ, which binds to the FixK2 promoter to induce transcription of the phage repressor cl. Repressor cl inhibits transcription from phage promoter pR, preventing expression of a reporter gene. When light is present, YFI is inactive, preventing repressor cl synthesis and allowing reporter gene transcription to take place.

When describing the embodiments of the present invention, the combinations and permutations of all possible embodiments have not been explicitly described. Nevertheless, the mere fact that certain measures are recited in mutually different dependent items or described in different embodiments does not indicate that a combination of these measures cannot be used to advantage. The present invention envisages all possible combinations and permutations of the described embodiments.

The terms "comprising", "comprise" and "comprises" herein are intended to be optionally substitutable with the terms "consisting of", "consist of" and "consist of", respectively, in every instance. The invention will be more fully understood by reference to the detailed description of the invention. This should not, however, be construed as limiting the scope of the invention. All literature citations are incorporated herein by reference in their entirety.

### EXAMPLE 1

The packaging cell line (Component 1) established in HEK293 cells and illustrated in Fig. 1 carrying the Gal-4 tag sequence between the ITRs and expressing the cap gene encoding the capsid of AAV2 was co-incubated overnight with the reporter cell line containing the firefly luciferase reporter-gene under the control of a 5-fold tandem repeat of the GAL4 UAS (Component 2) illustrated in Fig. 2 in the presence of a sample of human serum to be tested for the presence of neutralizing antibodies to the recombinant AAV2 vector. Firefly luciferase expression was then quantified in a luminometer using the BrightGlo substrate (Fig.6).

In specific embodiments, present invention relates to the following items;
1. A first metazoan cell (**Component 1**) for the production of the recombinant AAV vector, to which the antibody response is to be determined, comprising
   (i). the AAV genome or the transgene-promoter construct containing a tag sequence contained within the AAV ITRs
   (ii). the *cap* gene of the recombinant AAV vector, encoding either a naturally occurring Cap, hybrid, Cap or chimeric Cap operationally linked to a constitutive promoter
   (iii). the AAV *rep* gene, operationally linked to an inducible promoter
   (iv). the AAV *E2A, E4,* and *VA* genes, each operationally linked to individual natural or inducible promoters
   (v). the AAV *E1A* gene, operationally linked to an inducible promoter.
2. A second metazoan cell (**Component 2**) incorporating a reporter-gene that responds specifically to the tag sequence within the AAV genome or the transgene-promoter construct of **Component 1**
   and a method of using the same for detecting and optimally quantitating anti-AAV neutralizing antibodies in a test sample(s).
3. The cells of item 1 wherein the cell is a baculovirus infectable insect cell such as SF9 cells, an avian cell such as DT-40, MSB1, or LMH, a mouse cell such as L929 cells or the LS variant, a Chinese hamster ovary (CHO) cell such as CHO-K1, CHO-DXB11, CHO-DG44, CHOK1SV, including all variants, a CHOK1SV - GSKO (glutamine synthetase knock out) including all variants, a human cell such as HEK293 cells including all variants and all suspension or adherent variants, HeLa cells, HT1080 cells, ARPE-19, U937, Jurkat, HuH-7, HepG2, K562, A431, or A549 cells.
4. The cells of **Component 1** wherein the tag sequence contained within the AAV genome or the transgene-promoter construct is the Gal4 DNA binding domain
5. The cells of **Component 1** wherein the tag sequence contained within the AAV genome or the transgene-promoter construct encodes a cGMP specific receptor protein (CRP)
6. The cells of **Component 1** wherein the tag sequence contained within the AAV genome or the transgene construct encodes the trans-silencer VanR fused to the trans-activator VP16.
7. The cells of **Component 2** incorporating a reporter-gene operationally linked to a chimeric promoter comprising the Gal 4 upstream activation sequence (UAS) and a minimal promoter containing the transcriptional start point TATAA or variants thereof, or a tandem repeat of the Gal4 UAS, in a preferred embodiment a 5-fold tandem repeat thereof, that responds specifically to the gal4 tag sequence within the AAV genome or the transgene construct of **component 1.**
8. The cells of **Component 2** incorporating a reporter-gene operationally linked to a chimeric promoter comprising the GTA operator sequence and a minimal promoter, or a tandem repeat thereof, in a preferred embodiment a 4-fold tandem repeat thereof, that responds specifically to the CRP sequence within the AAV genome or the transgene-promoter construct of **component 1.**
9. The cells of **Component 2** incorporating a reporter-gene operationally linked to a chimeric promoter comprising a VanO operator module, in a preferred embodiment an octameric VanO operator module (Van0₈), and a minimal promoter such as the CMV immediate early promoter that responds specifically to the VanR sequence within the AAV genome or the transgene construct of **component 1.**
10. The cells of **Component 1** of any one of the preceding items wherein the cell comprises at least 5 recombinant target sites.
11. The cells of **Component 2** of any one of the items 1 to 4 wherein the reporter is a luciferase, such as firefly luciferase, Renilla luciferase, Metridia luciferase or other proteins including novel luciferases or fluorescent proteins such as green fluorescent proteins, enhanced green fluorescent protein, red fluorescent proteins, yellow fluorescent protein, blue fluorescent protein and variant there of displaying a different excitation/emission spectra.
   (i). In a preferred embodiment, to provide for a normalization of the assay, the cells of **Component 2** according to the present invention further have a construct for the constitutive expression of a luciferase that is different from that used in the reporter gene construct that responds to the tag sequence contained within the AAV genome or the transgene-promoter construct. For example, the constitutive production may be of a second luciferase, e.g., Renilla luciferase, firefly luciferase or Metridia luciferase.
12. A method for detecting and optionally quantitating the activity of an anti-AAV neutralizing antibody in a test sample, said method comprising the steps of
   (i) providing a test sample(s), thought to contain anti-AAV antibodies together with control sample(s) without anti-AAV antibodies and in a preferred embodiment a positive control sample(s) known to contain anti-AAV neutralizing antibodies.
   (ii) contacting said test sample(s) with either the virus produced by the cells of **Component 1,** or the virus-producing cells of **Component 1** according to any one of the preceding items,
   (iii) incubating said test sample(s) with either the virus produced by the cells of **Component 1,** or the virus-producing cells of **Component 1** according to any one of the preceding items with the cells of **Component 2** at various temperatures preferably 37°C for various times preferable from 6 to 18 hours or more prior to determining the activity of the AAV-tag responsive first reporter protein in said cell line.
13. The method according to any of the preceding items, wherein said Reporter cell line expresses a second reporter protein, said method further comprising
   (iv) determining the activity of the first reporter protein in said cell line,
   (v) providing the ratio between the activity of the first reporter protein and the second reporter protein.
   (vi) determining the activity of the first tag-responsive reporter protein in the reporter cells of the first cell sample normalized or not relative to the activity of the second luciferase is described in US 2011/0189658
   (vii) and determining the activity of the first tag-responsive reporter protein in the cells of the second control cell sample, normalized or not relative to the activity of the second luciferase is described in US 2011/0189658.
   (viii) provide the ratio between the reporter activity in first and a second control cell sample, where a ratio (first/second) lower than one is indicative for the presence of antibodies against the AAV serotype or recombinant AAV vector in said sample.
14. A method for high throughput screening of patients samples for detecting and optimally monitoring the immune response to either the AAV capsid, AAV genome, transgene, or transgene product of a recombinant AAV vector said method comprising the steps of
   (i) providing a test sample(s), consisting of patients samples to be screened
   (ii) contacting said test sample(s) with the challenge virus or virus-producing packaging cells of **Component 1** according to the present invention and according to any one of the preceding items,
   (iii) incubating said test sample(s) with the challenge virus or virus-producing packaging cells of **Component 1** according to any one of the preceding items with the cells of **Component 2** at various temperatures preferably 37°C and for various times preferably from 6 to 18 hours or more prior to determining the activity of the AAV-tag responsive first reporter protein in said cell line.
      The method according to any of the preceding items, wherein said cell line expresses a second reporter protein, said method further comprising
   (iv) determining the activity of the first tag-responsive reporter protein in said cell line,
   (v) providing the ratio between the activity of said cell line, **Component 2**, containing a first heterologous polynucleotide comprising a heterologous cis-acting regulatory sequence, that responds to treatment of the cell line with the tag sequence present in the genome of the AAV serotype or recombinant AAV vector construct, operably linked to a downstream promoter sequence, wherein said promoter is operably linked to an open reading frame encoding a first reporter protein such as a luciferase, e.g., Renilla luciferase, firefly luciferase or Metridia luciferase.
   (vi) in a preferred embodiment, to provide for a normalization of the assay, the cells of **Component 2** according to the present invention further have a construct for the constitutive expression of a luciferase that is different from that used in the reporter gene construct that responds to the tag sequence contained within the AAV genome or the transgene construct. For example, the constitutive production may be of a second luciferase, e.g., Renilla luciferase, firefly luciferase, or Metridia luciferase.
   (vii) in a preferred embodiment the cells according to the present invention, are seeded in 96, 384 or 1536 well plates assay plates.

### References

1. Fitzpatrick Z. et al. Mol Therapy: Methods & Clinical Develop. 9:119-129, 2018
2. Colella, P. et al. Molecular Therapy. 8:87-104, 2018.
3. Lee, EJ., Guenther, CM., Suh, J (2018). Curr. Opin., Biomed. Eng. 7:58-63
4. Muzcyzka, N. (1992).. Curr. Top. Microbiol. Immunol. 158:97-129.
5. Buller RM, et al. J. Virol. 40 :241-247, 1981
6. Schlehofer, JR., et al. Virology. 152:110-117, 1986.
7. King, JA., et al EMBO J. 20:3282-3291. (2001).
8. Agbandje-McKenna, M. & Kleinschmidt, in Adeno-associated Virus 47-92, 2012
9. Pillay, S., et al. Nature 530: 108-112, 2016
10. Dudek, AM. Molecular Therapy. 28:1-15, 2019.
11. Li, J., Samulski, RJ., & Xiao, X. J. Virol. 71:5236-5243, 1997
12. Xiao, X., Li, J., and Samulski, RJ. J. Virol. 72:2224-2232, 1998
13. Allen, JM., et al. Mol. Therapy. 1:88-95. 2000
14. Li, C., and Samulski, RJ. Nat. Rev. Gen.. 21:255-272, 2020.
15. Lallemand C. A J Immunol Res. 390:1-19, 2017

## Claims

1. Use of two cell lines in a diagnostic method, wherein one cell line is referred to as Component 1 comprising:
i). the AAV genome or the transgene-promoter construct comprising one or more tag sequences contained within the AAV ITRs and wherein the tag sequences may be the same or different from each other,
(ii). the cap gene of a recombinant AAV vector, encoding either a naturally occurring Cap, hybrid, Cap, or chimeric Cap operationally linked to a constitutive promoter or inducible promotor,
(iii). the AAV rep gene, operationally linked to a natural promoter or an inducible promoter,
(iv). the AAV *E2A, E4, and VA* genes, each operationally linked to individual natural or inducible promoters,
(v). the AAV *E1A* gene, operationally linked to a natural or an inducible promoter,
and a further cell line referred to as Component 2 comprising:
comprising one or more reporter-genes that respond specifically to the one or more tag sequences according to (i) within the AAV genome or the transgene-promoter construct of the cell in Component 1.

2. Use according to claim 1, wherein the constitutive promotor is selected cytomegalovirus (CMV) early enhancer/promoter, SV40 promoter, UBC promoter, PGK promoter, human β-actin (hACTB), human elongation factor-1a (hEF-1α), Thymidine Kinase (TK) promoter and cytomegalovirus early enhancer/chicken β-actin (CAG) promoters.

3. Use according to any one or the preceding claims, wherein the inducible promotor is selected from the Tet-on/Tet-off system, the cumate-inducible repressor CymR system, the flavonoid phloretin regulated TtgR repressor system, and the vanillic acid regulated VanR repressor/KRAP system, and wherein the inducible promotor in (iii), (iv), (v) are different from each other.

4. Use according to any of the preceding claims, wherein cells in Component 1 expresses a transgene-promoter construct containing a tag sequence encoding the Gal4 DNA binding domain, that will bind specifically to the Gal4 upstream activation sequence (UAS) regulating expression of the firefly luciferase (FL) gene, or expresses the VanR sequence within the AAV genome or the transgene construct, or the AAV genome or the transgene-promoter construct encoding a cGMP specific receptor protein (CRP).

5. Use according to any of the preceding claims, wherein the tag sequence contained within the AAV genome or the transgene construct encodes the trans-silencer VanR fused to the trans-activator VP16.

6. Use according to any of the preceding claims, wherein Component 2 comprises a reporter-gene operationally linked to a chimeric promoter comprising the Gal 4 upstream activation sequence (UAS) and a minimal promoter containing the transcriptional start point TATAA or variants thereof, or a tandem repeat of the Gal4 UAS, or optionally a 5-fold tandem repeat thereof, that responds specifically to the gal4 tag sequence within the AAV genome or the transgene construct of Component 1.

7. Use according to any of the preceding claims, wherein Component 2 comprises a reporter-gene operationally linked to a chimeric promoter comprising the GTA operator sequence and a minimal promoter, or a tandem repeat thereof, or a 4-fold tandem repeat thereof, that responds specifically to the CRP sequence within the AAV genome or the transgene-promoter construct of Component 1, and wherein the reporter reporter denoted as the first reporter protein is a luciferase, such as firefly luciferase, Renilla luciferase, Metridia luciferase or other proteins including novel luciferases or fluorescent proteins such as green fluorescent proteins, enhanced green fluorescent protein, red fluorescent proteins, yellow fluorescent protein, blue fluorescent protein and variant there of displaying a different excitation/emission spectra.

8. Use according to any of the preceding claims, wherein Component 2 comprises a reporter-gene operationally linked to a chimeric promoter comprising a VanO operator module, or an octameric VanO operator module (Van08), and a minimal promoter such as the CMV immediate early promoter that responds specifically to the VanR sequence within the AAV genome or the transgene construct of Component 1.

9. Use according to any of the preceding claims, wherein Component 2 further comprises a construct for the constitutive expression of a luciferase that is different from that used in the reporter gene construct that responds to the one or more tag sequence contained within the AAV genome or the transgene construct in Component 1 and is e.g. the constitutive production of a second luciferase, e.g., Renilla luciferase, firefly luciferase, or Metridia luciferase.

10. Use according to any of the preceding claims, Component 1 comprises at least 5 recombinant target sites.

11. Use according to any of the preceding claims, wherein the diagnostic method comprises detection and quantification of neutralizing antibodies directed against recombinant adeno-associated virus (AAV) vectors, or in high throughput screening of patients samples for detecting and optimally monitoring the immune response to either the AAV capsid, AAV genome, transgene, or transgene product by the aid of both Component 1 and Component 2.

12. A method for detecting and optionally quantitating the activity of an anti-AAV neutralizing antibody in a test sample, the method comprising the steps of;
(i) providing a test sample(s), thought to contain anti-AAV antibodies together with control sample(s) without anti-AAV antibodies and optionally also a positive control sample(s) known to contain anti-AAV neutralizing antibodies,
(ii) contacting said test sample(s) in (i) with either the virus produced by the cells in Component 1 according to any one of the preceding claims, or the virus-producing cells of Component 1 according to any one of the preceding claims, or alternatively incubating said test sample(s) with either the virus produced by the cells in Component 1 according to any one of the preceding claims, or the virus-producing cells in Component 1 according to any one of the preceding claims, with the cells of Component 2 at various temperatures preferably about 37°C for various times preferable from about 6 to about 18 hours or more prior to determining the activity of the AAV-tag responsive first reporter protein in the cells of Component 2.

13. The method according to claim 12, wherein the cells of Component 2 expresses a second reporter protein which is different from the first tag reporter protein, said method further comprising the steps of;
(iv) determining the activity of the first tag reporter protein in the cells of Component 2,
(v) providing the ratio between the activity of the first tag reporter protein and the second reporter protein in Component 2,
(vi) determining the activity of the first tag-responsive reporter protein in the said cells of the first cell sample normalized or not relative to the activity of the second reporter protein,
(vii) determining the activity of the first tag-responsive reporter protein in the cells of the second control cell sample, normalized or not relative to the activity of the second reporter protein,
(viii) provide the ratio between the reporter activity in first and a second control cell sample, where a ratio (first/second) lower than one is indicative for the presence of antibodies against the AAV serotype or recombinant AAV vector in said sample.

14. A method for high throughput screening of patients samples for detecting and optimally monitoring the immune response to either the AAV capsid, AAV genome, transgene, or transgene product said method comprising the steps of;
(iv) providing a test sample(s), consisting of patients samples to be screened,
(v) contacting said test sample(s) with the challenge virus or virus-producing cells of Component 1 specified in any one of claims 1-11,
(vi) incubating said test sample(s) with the challenge virus or virus-producing cells of Component 1 specified in any one of claims 1-11 with the cells of Component 2 specified in any one of claims 1-11 at various temperatures preferably about 37°C and for various times preferably from about 6 to about 18 hours or more prior to determining the activity of the AAV-tag responsive first reporter protein in said cell line.

15. The method according to claim 14, wherein said cells of Component 2 as specified in any one of claims 1-11 further comprises a second reporter protein which is different from the first tag reporter protein , said method further comprising the steps of;
(iv) determining the activity of the first tag-responsive reporter protein in the cells of Component 2 as specified in any one of claims 1-11,
(viii) providing the ratio between the activity of the cells of Component 2 as specified in any one of claims 1-11, comprising a first heterologous polynucleotide comprising a heterologous cis-acting regulatory sequence, that responds to treatment of the cell line with the tag sequence present in the genome of the AAV serotype or recombinant AAV vector construct in Component 1, operably linked to a downstream promoter sequence, wherein said promoter is operably linked to an open reading frame encoding a first reporter protein such as a luciferase, e.g., Renilla luciferase, firefly luciferase or Metridia luciferase.
(ix) optionally, to provide for a normalization of the assay, the cells of Component 2 as specified in any one of claims 1-11 further comprises a construct for the constitutive expression of a luciferase that is different from that used in the reporter gene construct that responds to the tag sequence contained within the AAV genome or the transgene construct in Component 1, such as e.g. the constitutive production is a second luciferase, e.g., Renilla luciferase, firefly luciferase, or Metridia luciferase.
